# EUROPÄISCHE PATENTSCHRIFT

(11) **EP 1 464 300 B1**
(45) Veröffentlichungstag und Bekanntmachung des Hinweises auf die Patenterteilung: **29.10.2008**
(21) Anmeldenummer: 03005675.8
(22) Anmeldetag: 13.03.2003
(51) Int. Cl.: A61F 2/06, A61F 2/04

(54) **Blutgefäss-Urethrastent**
Blood vessel-urethra stent
Vaisseaux sanguins-uretre stent

(43) Veröffentlichungstag der Anmeldung: 06.10.2004
(73) Patentinhaber: Reflow GmbH, 83053 Bruckmühl (DE)
(72) Erfinder: Walter, Thomas, 23970 Wismar (DE); Meyer, Andreas, 23970 Wismar (DE)
(74) Vertreter: Hansen, Jochen

(56) Entgegenhaltungen:
- EP-A- 0 943 299
- WO-A-00/44308
- FR-A- 2 767 673
- US-A- 5 167 614
- US-A- 5 766 209
- US-A1- 2002 143 388

## Beschreibung

Die Erfindung betrifft einen Stent zur Aufrechterhaltung des Volumenstromes in Blutgefäßen oder in der Urethra, insbesondere zum temporären Abstützen der Prostata, bestehend aus einem dünnwandigen, im wesentlichen rohrförmigen Körper mit einer Einlauföffnung an einem Ende und einer Auslauföffnung am anderen Ende.

Derartige Stents sind zur Aufrechterhaltung des Volumenstromes zwischen Harnblase und Harnausgang in unterschiedlichen Ausgestaltungen im Bereich der Medizintechnik bekannt. Dabei ist für derartige Urethrastents typisches Anwendungsgebiet das Offenhalten der prostatischen Harnröhre, beispielsweise bei benigner Prostatahyperplasie. Urethrastents werden minimalinvasiv mit Hilfe eines Zystoskopes transurethral implantiert. Ein weiteres Anwendungsgebiet derartiger Stents ist das Offenhalten von peripheren Blutgefäßen oder zur Stützauskleidung von Aortenaneurysmen.

Für beide Anwendungsbereiche sind die bekannten Stents aus Metallgitterrohren gebildet, die nach dem Setzen im Blutgefäß bzw. in der Harnröhre in situ belassen werden. Insbesondere in Blutgefäßen, beispielsweise in kardialen Gefäßen, wachsen diese Metallgitterrohre im Gefäßgewebe ein. Eine spätere Entfernung ist daher unmöglich, was insbesondere Schwierigkeiten bereitet, wenn im Anschluss an einen gestützen Gefäßbereich unmittelbar anschließend eine weitere Gefäßverengung auftritt und folglich hier ebenfalls ein Stützmetallgitterrohr zu setzen ist. Dies ist technisch kompliziert oder gar unmöglich.

Ferner ist nachteilig beim Einsatz in der Harnröhre, dass die bekannten Urethrastents bei längerer Einsatzzeit, eine Blutungs- und/oder Infektionsgefahr und insbesondere eine Zellproliferation mit nachfolgender Volumenrestriktion hervorrufen. Darüber hinaus besteht häufig eine schlechte Toleranz gegenüber dem im Körper eingesetzten Stent als Fremdkörper. Ein Entfernen eines in der Harnröhre gesetzten Metallgitterrohr-Stent bedeutet für den Patienten eine erhebliche Belastung als invasiven Eingriff.

Aus der nachveröffentlichten DE 101 55 767 ist eine Ureterschiene zur Aufrechterhaltung eines Volumenstromes und zum temporären Abstützen der Harnröhre bekannt, bei dem ein flexibler, im wesentlichen rohrförmiger Schienenkörper mit einer Vielzahl von in der Mantelfläche des Schienenkörpers vorgesehenen Öffnungen ausgebildet ist und aus einem im Patienten resorbierbaren Material gebildet ist. Diese Ureterschiene unterstützt die Drainage der Niere und Ableitung des Harns zur Harnblase. Durch das resorbierbare Material löst sich der Schienenkörper nach Erfüllung der therapeutischen Funktion im Patienten auf. Eine zusätzliche Patientenbelastung zur Entfernung der Ureterschiene wird hier ebenfalls vermieden. Die hier vorgeschlagene Ureterschiene ist jedoch für den Einsatz als Urethrastent oder als Blutgefäßstent nicht geeignet, da die in der Mantelfläche vorgesehenen Öffnungen einer bevorzugten ganzflächigen Stützwirkung widersprechen. Zudem besteht die Gefahr, dass an den Öffnungen Einwachsungen entstehen, die wiederum eine durchflussvermindernde Verengung hervorrufen können.

Ferner ist aus der WO 00/44308 eine Intravaskulärvorrichtung sowie ein Herstellungsverfahren dafür bekannt, bei dem der Stützkörper aus fadenförmigem Material gewoben ist. Das fadenförmige Material kann auch resorbierbar ausgebildet sein. Dabei handelt es sich um einen selbstexpandierbaren oder ballonexpandierbaren Stent. Insbesondere ist das fadenförmige Material aus Formgedächtnisfilamenten gebildet, so dass er sich entsprechend einer voreingeprägten Form selbst aufweitend ausgebildet ist.

Aus der US 2002/1043388 A1 ist ein biologisch abbaubarer Stent bekannt, der auch rohrförmig ausgebildet sein kann. Als Material wird ein abbaubares Polymer bevorzugt.

Die EP 0 943 299 A1 beschreibt einen Stent gemäß der Präambel des Anspruchs 1, der eine balgenartige oder Faltenschlauchausbildung zeigt.

Aus der US 5,766,209 ist ein Urethrastent aus optional resorbierbarem Material mit einer speziellen Ausbildung zur Funktionserhaltung des Schließmuskels bekannt. Der Stentkörper ist in diesem Bereich mehrfach in axialer Richtung geschlitzt.

Aufgabe der Erfindung ist es, einen Stent anzugeben, der die Patientenbelastung verringert, ggf. ein erneutes Setzen eines Stents an gleicher oder nur geringfügig versetzter Position ermöglicht und in seiner Lage fixiert ist.

Gelöst wird diese Aufgabe mit einem Stent gemäß Anspruch 1.

Durch die Ausbildung des rohrförmigen Körpers als geschlossene Mantelfläche wird die Belastung der Blutgefäß- bzw. Urethrawandung deutlich verringert. Ferner wird eine unerwünschte Inkrustation oder Einwachsung des Stents im Wandungsgewebe vermieden. Die Ausbildung des Stents aus einem biokompatiblen, resorbierbaren Material erlaubt die vollständige Auflösung des im betreffenden Hohlorgan des Patienten intubierten Stents nach Erfüllung seiner therapeutischen Funktion. Nach Resorption des Stents ist weder eine invasive Extubation erforderlich noch ein möglicherweise unerwünschtes Zurückbleiben des Stents als Fremdkörper im Patienten zu berücksichtigen. Bevorzugt besteht der Stent aus einem flexiblen Material, um Reizungen beim Patienten weiter zu verringern.

An der Mantelfläche des Stents sind nach außen gerichtete Fixierungselemente vorgesehen, wodurch eine Dislozierung des im Hohlorgan eingezetzten Stents verhindert wird. Dadurch, dass die Fixierungselemente elastisch federnd abspreizend and der Mantelfläche des Stents angeformt sind, kann der Stent beispielsweise durch herkömmliches Zystoskop an der gewünschten Einsatzstelle ausgebracht werden, wobei beim Herausschieben die elastich federnd abspreizenden Fixierungselemente im Hohlorgan an dessen Wandung Halt finden und den Stent in axialer Richtung fixieren.

Dadurch, dass nahe der Einlauföffnung und nahe der Auslauföffnung je drei um 120° in radialer Ebene zum Stent angeordnete Fixierungselemente vorgesehen sind, wobei beide Fixierungselementanordnungen gegenläufige axiale Bewegungsrichtungen des Stents blockieren, wird eine besonders zuverlässige Festlegung des Stents im Blutgefäß bzw. in der Harnröhre erreicht.

Ferner kann in Folge der Resorption des Urethrastents ein dauerhaftes Einwachsen in ein vorgeschädigtes Harnröhrengewebe verbunden mit einer fremdkörperinduzierten Harnröhrenobstruktion vermieden werden.

Wenn im resorbierbaren Material laserlichtreflektierende Kleinstpartikel im wesentlichen gleichmäßig verteilt aufgenommen sind, wird eine etwa im Einzelfall notwendige Laserablation durch Reflektion des Laserlichts unterstützt und ermöglicht den Einsatz mit geringerer Laserenergie.

Dadurch, dass im resorbierbaren Material und/oder auf dessen Oberflächen inkrustations- und/oder entzündungshemmende Pharmaka im wesentlichen gleichmäßig verteilt angeordnet sind, kann ein Einwachsen des Stents in ein vorgeschädigtes Wandungsgewebe des Patienten besonders zuverlässig vermieden werden. Alternativ und ergänzend können durch im resorbierbaren Material eingelagerte Pharmaka Entzündungen gehemmt werden. Die Pharmaka sind bevorzugt gleichmäßig verteilt im resorbierbaren Material eingebettet. Bei Resorption des Stentmaterials werden die Pharmaka an Ort und Stelle dosiert freigesetzt. Es können somit die therapeutische Funktion des Stents unterstützende Medikamente oder therapeutische Begleitmedikamente wohl dosiert zugeführt werden. Durch die Freisetzung der Medikamentenwirkstoffe unmittelbar am zu behandelnden Organ kann eine gleichmäßige Langzeitdosierung mit zielgenauer Wirkstofffreigabe während des gesamten Resorptionsprozesses des Stents erreicht werden. Die Wirkstoff-Freigabe wird minimal dosiert, womit etwaige Nebenwirkungen reduziert werden. Bevorzugte Pharmaka sind Zytostatika bei malignen Erkrankungen; Antibiotika, wie z. B. Fluorchinolane und Nitroxilin; Antimykotika, wie z. B. Fluconazol; Analgetika wie z. B. Lidokain; und Spasmolytika, wie z. B. Butylscopolaminiumbromid, die als Einzelsubstanz oder in der Summe enthalten sind.

Wenn Röntgen-, CT- und/oder NMR-Kontrastmittel im resorbierbaren Material im wesentlichen gleichmäßig verteilt eingebettet sind, kann die Lage, der Resorptionsprozess und auch die therapeutische Funktion des im Blutgefäß oder in der Harnröhre angeordneten Stents durch entsprechende Untersuchungsverfahren per Röntgenaufnahme, Computertomographie oder NMR-Resonanzbild festgestellt werden. Die Kontrastmittel werden bei Resorption des Stentmaterials über natürliche Stoffwechselfunktionen ausgeschieden bzw. mit dem Harn ausgespült.

Wenn Metallionen im resorbierbaren Material des Stents eingebettet sind, kann eine thermoinduzierte Entfernung des Stents erreicht werden. Die eingelagerten Metallionen erhöhen die Leitfähigkeit des Stents, so dass bei Anlegen einer elektrischen Spannung an den Stent mittels einer im Gefäß bzw. in der Urethra eingebrachten Elektrosonde eine thermoinduzierte Auflösung des Stentmaterials hervorgerufen werden kann.

Dadurch, dass die geschlossene Mantelfläche außenseitig mikroporös ausgebildet ist, wird einerseits die Einlagerung von Kontrastmitteln, Metallionen und/oder Pharmaka erleichtert und andererseits ebenso die Abgabe dieser Teilchen.

Als resorbierbares Material für den Stent eignet sich insbesondere Poly-(L)-Lactid, Poly-(DL)-Lactid, Polyglycolsäure, Polymere der Maleinsäure, Poly-β-Hydroxybuttersäure, Polydioxanon, Blends oder Copolymeren aus diesen.

Um eine möglichst große Flexibilität des aus resorbierbarem Material hergestellten Stents zu erreichen und damit die Toleranz des Fremdkörpers durch den Patienten zu verbessern, sind dem resorbierbaren Material biokompatible Weichmacher zugesetzt.

Für die Anwendung als Urethrastent ist es bevorzugt, dass an der der Harnblase zugewandten Einlauföffnung eine trichter- oder schirmartige Aufweitung vorgesehen ist. Die trichter- oder schirmartige Aufweitung oder Ausstülpung des Urethrastent fixiert sich am Blasenhals, womit ein Verschieben des Urethrastents vermieden wird und der Übergangsbereich von der Blase zur Prostata mit der gewünschten Öffnungsweite freigehalten wird. Bevorzugt ist die Aufweitung flächig und biegeschlaff ausgebildet.

Wenn die trichter- oder schirmartige Aufweitung wenigstens einen im wesentlichen radialen Einschnitt aufweist, kann die Aufweitung oder Ausstülpung wie ein Segel innerhalb eines herkömmlichen Zystoskopes zusammengelegt werden und der Urethrastent transurethral mit seiner zusammengelegten Ausstülpung bis in den Blasenhals geführt werden. Erst beim Herausschieben des Stents aus dem Zystoskop entfaltet sich die Aufweitung. Alternativ oder ergänzend kann für diesen Zweck auch die trichter- oder schirmartige Aufweitung wenigstens einen im wesentlichen kreissegmentierten Ausschnitt aufweisen.

Als Urethrastent weist der Stentkörper einen Außendurchmesser von 4 bis 7 mm, bevorzugt 5 mm, sowie eine Länge von 80 bis 120 mm, bevorzugt 100 mm auf.

Nachfolgend werden zwei Ausführungsbeispiele der Erfindung anhand der beiliegenden Zeichnungen detalliert beschrieben.

Darin zeigt:
- Fig. 1: in einer teils geschnittenen Ansicht einen erfindungsgemäßen Stent in einem Blutgefäß,
- Fig. 2: in einer teils geschnittenen Ansicht einen in der Urethra gesetzten Stent mit umliegenden Organen,
- Fig. 3: den in Fig. 2 dargestellten Stent im Längsschnitt und
- Fig. 4: in einer Draufsicht eine schirmartige Aufweitung des Urethrastents gemäß der Fig. 2 und 3.

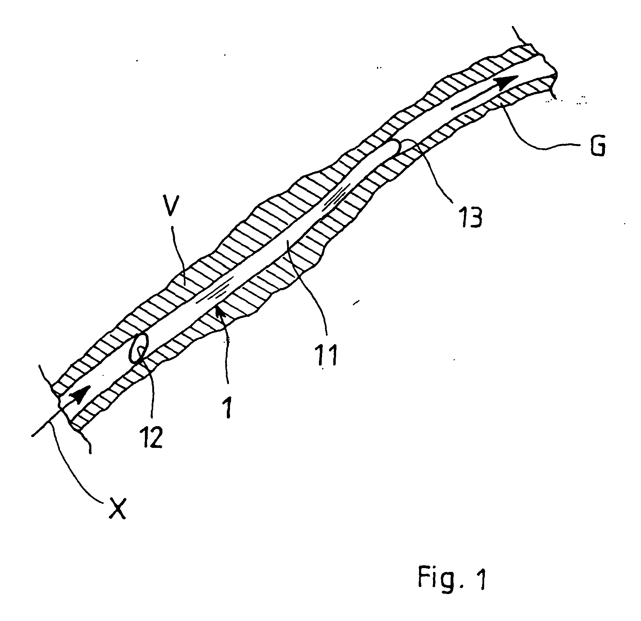

In Fig. 1 ist ein Blutgefäß G im Schnitt dargestellt. Im Bereich einer Gefäßverengung V ist ein Stent 1 bestehend aus einem flexiblen, dünnwandigen, im wesentlichen rohrförmigen Körper eingesetzt. Der rohrförmige Körper besteht aus einer geschlossenen Mantelfläche 11 aus einem im Patienten resorbierbaren Material. An einem Ende des rohrförmigen Körpers 11 ist eine Einlauföffnung 12 und am anderen Ende eine Auslauföffnung 13 angeordnet.

Das im Blutgefäß G transportierte Blut wird in Strömungsrichtung X über Einlauföffnung 12 im rohrförmigen Körper 11 zur Auslauföffnung 13 unter Offenhaltung der Gefäßverengung V geführt.

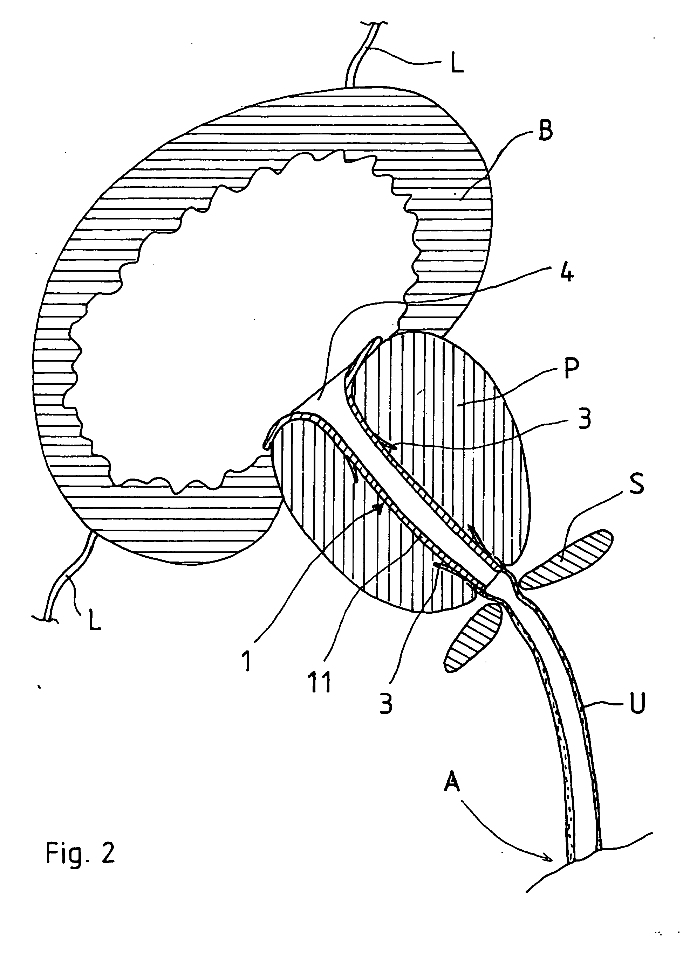

In Fig. 2 sind als Prinzipskizze die inneren Organe im Bereich der Urethra dargestellt. Zwei Harnleiter (Ureter) L leiten den von den nicht dargestellten Nieren abgegebenen Harn zur Harnblase B. Die Harnblase B entleert über die Harnröhre (Urethra) U zum Harnausgang A. Die Urethra U ist im Bereich zwischen Harnblasenhals H der Harnblase B und einem Schließmuskel S von der Prostata P umgeben.

Zur Aufrechterhaltung des Volumenstromes durch die Urethra ist im Bereich der Prostata ein Stent 1 eingesetzt. Der Stent 1 ist über den Harnausgang A urethral in die Harnröhre U mit einem Zystoskop eingeführt worden.

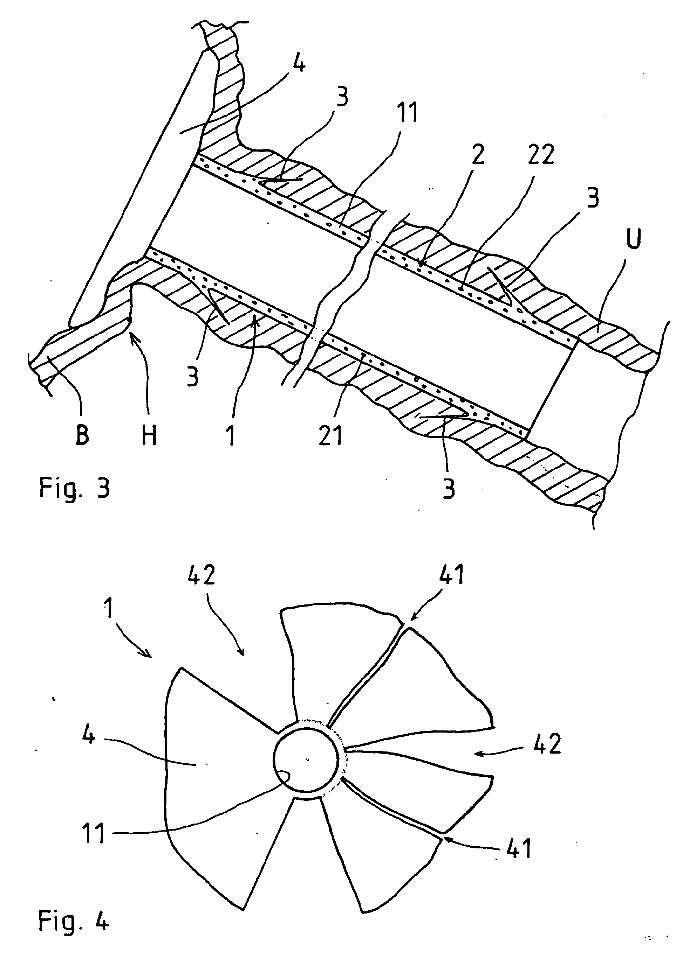

Der Urethrastent 1 besitzt an seinem distalen Ende, also der dem Harnblasenhals H zugewandten Einlauföffnung 12 des Stents 1 eine trichter- oder schirmartige Aufweitung oder Ausstülpung 4, die den Übergangsbereich zwischen Harnblasenhals H und der Urethra U im Bereich der Prostata P stützt. Wie in Fig. 4 dargestellt, ist die trichter- oder schirmartige Aufweitung 4 annähernd in einer Radialfläche zur Stentachse flächig ausgebildet.

Um einerseits eine gute Anschmiegbarkeit der Ausstülpung 4 an das zu stützende Hohlorgan zu gewährleisten und andererseits eine Faltbarkeit innerhalb des Zystoskops zur problemlosen Einführung des Stents sicherzustellen, sind Einschnitte 41 und/oder Ausschnitte 42 in der flächigen, biegeschlaff ausgebildeten Aufweitung 4 ausgeführt. Die Einschnitte 41 sind dabei radial zur Stentachse ausgebildet. Die Ausschnitte 42 sparen Material der flächigen Aufweitung 4 aus, so dass kreissegmentförmige Materialabschnitte als Ausstülpung 4 übrigbleiben. Diese so gebildete Aufweitung bzw. Ausstülpung 4 wird wie ein Segel zusammengefaltet im Zystoskop aufgenommen. Beim Herausdrücken des Stents 1 an der vorgesehenen Position entfaltet sich die Ausstülpung 4 selbsttätig und legt sich um den Blasenhals H und Eingang der Urethra U im Bereich der Prostata P an das Hohlorgan an. Diese Ausstülpung 4 liefert damit gleichzeitig eine Lagefixierung des Stents 1 innerhalb des Hohlorgans.

Eine weitere Lagefixierung des Stents 1 innerhalb der Harnröhre U wird durch an der Mantelfläche 11 des rohrförmigen Stentkörpers 1 elastisch federnd abspreizende Fixierungselemente 3 sichergestellt, wie sie in Fig. 2 und 3 dargestellt sind. Die Fixierungselemente 3 sind bevorzugt nahe der Einlauföffnung 12 bzw. nahe der Auslauföffnung 13 angeordnet. Bevorzugt sind je Fixierungselementanordnung drei Fixierungselemente 3 um 120° in radialer Ebene zum Stent 1 angeordnet. Die federnd abspreizenden Fixierungselemente 3 bilden je axialer Bewegungsrichtung des Stents 1 eine Lagefixierung in Art eines Widerhakens.

Beim Einführen des Stents 1 sind die Fixierungselemente 3 innerhalb des Zystoskops an die Mantelfläche 11 des Stents 1 angeklappt. Erst nach Herausschieben des Stents 1 aus dem Zystoskop spreizen die Fixierungselemente 3 von der Mantelfläche 11 ab und verankern sich im Gewebe der Harnröhre U.

Ferner ist in Fig. 3 im Querschnitt das Material der dünnwandigen Mantelfläche 11 dargestellt. In diesem resorbierbaren Material sind Partikel 2 im wesentlichen gleichmäßig verteilt eingebettet. Im dargestellten Ausführungsbeispiel sind laserlichtreflektierende Kleinstpartikel 21 sowie Arzneimittelpartikel 22 in dem Material eingebettet. Die Wandstärke aus resorbierbarem Material des rohrförmigen Körpers 11 ist zeichnerisch gegenüber seinem Durchmesser vergrößert dargestellt, um die im resorbierbaren Material eingebetteten Partikel visualisieren zu können. Die typische Wandstärke der Stentmantelfläche beträgt 0,5 mm.

### Bezugszeichenliste

- 1: Stent
- 11: rohrförmiger Körper, Mantelfläche
- 12: Einlauföffnung
- 13: Auslauföffnung

- 2: Partikel
- 21: laserlichtreflektierende Partikel
- 22: Arzneimittelpartikel

- 3: Fixierungselement

- 4: Aufweitung, Ausstülpung
- 41: Einschnitt
- 42: Ausschnitt

- A: Harnausgang
- B: Harnblase
- G: Blutgefäß
- H: Harnblasenhals
- L: Harnleiter, Ureter
- P: Prostata
- S: Schließmuskel
- U: Harnröhre, Urethra
- V: Gefäßverengung
- X: Strömungsrichtung

## Patentansprüche

1. Stent (1) zur Aufrechterhaltung des Volumenstromes in Blutgefäßen (G) oder in der Urethra (U), insbesondere zum temporären Abstützen der Prostata (P), bestehend aus einem dünnwandigen, im wesentlichen rohrförmigen Körper mit einer Einlauföffnung (12) an einem Ende und einer Auslauföffnung (13) am anderen Ende, wobei der Körper aus einem im Patienten resorbierbaren Material gebildet ist, wobei der Körper eine geschlossene Mantelfläche (11) aufweist und an der Mantelfläche des Stents (1) nach außen gerichtete Fixierungselemente vorgesehen sind, **dadurch gekennzeichnet, dass** die Fixierungselemente elastisch federnd abspreizend an der Mantelfläche (11) des Stents (1) angeformt sind.

2. Stent nach Anspruch 1, **dadurch gekennzeichnet, dass** nahe der Einlauföffnung (12) und nahe der Auslauföffnung (13) je drei um 120° in radialer Ebene zum Stent (1) angeordnete Fixierungselemente (3) vorgesehen sind, wobei beide Fixierungselementanordnungen gegenläufige axiale Bewegungsrichtungen des Stents (1) blockieren.

3. Stent nach Anspruch 1 oder 2, **dadurch gekennzeichnet, dass** im resorbierbaren Material laserlichtreflektierende Kleinstpartikel (21) im wesentlichen gleichmäßig verteilt aufgenommen sind.

4. Stent nach Anspruch 1, 2 oder 3, **dadurch gekennzeichnet, dass** im resorbierbaren Material und/oder auf dessen Oberflächen inkrustations- und/oder entzündungshemmende Pharmaka im wesentlichen gleichmäßig verteilt angeordnet sind.

5. Stent nach Anspruch 4, **dadurch gekennzeichnet, dass** als Pharmaka Antibiotika wie z. B. Fluorchinolane und Nitroxilin, Antimykotika wie z. B. Fluconazol, Analgetika wie z. B. Lidokain und Spasmolytika wie z. B. Butylscopolaminiumbromid als Einzelsubstanz oder in der Summe enthalten sind.

6. Stent nach einem der vorangehenden Ansprüche, **dadurch gekennzeichnet, dass** Röntgen-, CT- und/oder NMR-Kontrastmittel im resorbierbaren Material im wesentlichen gleichmäßig verteilt eingebettet sind.

7. Stent nach einem der vorangehenden Ansprüche, **dadurch gekennzeichnet, dass** Metallionen (22) im resorbierbaren Material des Stents (1) eingebettet sind.

8. Stent nach einem der vorangehenden Ansprüche, **dadurch gekennzeichnet, dass** die geschlossene Mantelfläche (11) außenseitig mikroporös ausgebildet ist.

9. Stent nach einem der vorangehenden Ansprüche, **dadurch gekennzeichnet, dass** das resorbierbare Material aus Poly-(L)-Lactid, Poly-(DL)-Lactid, Polyglycolsäure, Polymeren der Maleinsäure, Poly-β-Hydroxybuttersäure, Polydioxanon, Blends oder Copolymeren aus diesen gebildet ist.

10. Stent nach einem der vorangehenden Ansprüche, **dadurch gekennzeichnet, dass** dem resorbierbaren Material biokompatible Weichmacher zugesetzt sind.

11. Stent zur Aufrechterhaltung des Volumenstroms in der Urethra (U) zwischen Harnblase (B) und Harnausgang (A) nach einem der vorangehenden Ansprüche, **dadurch gekennzeichnet, dass** an der der Harnblase (B) zugewandten Einlauföffnung (12) eine trichter- oder schirmartige Aufweitung (4) vorgesehen ist.

12. Stent nach Anspruch 11, **dadurch gekennzeichnet, dass** die Aufweitung (4) flächig und biegeschlaff ausgebildet ist.

13. Stent nach Anspruch 11 oder 12, **dadurch gekennzeichnet, dass** die trichter- oder schirmartige Aufweitung (4) wenigstens einen im wesentlichen radialen Einschnitt (41) aufweist.

14. Stent nach Anspruch 11, 12 oder 13, **dadurch gekennzeichnet, dass** die trichter- oder schirmartige Aufweitung (4) wenigstens einen im wesentlichen kreissegmentierten Ausschnitt (42) aufweist.

15. Stent nach einem der Ansprüche 11 bis 14, **dadurch gekennzeichnet, dass** der Stentkörper einen Außendurchmesser von 4 bis 7 mm, bevorzugt 5 mm, sowie eine Länge von 80 bis 120 mm, bevorzugt 100 mm aufweist.

## Claims

1. A stent (1) for maintaining the volume flow in blood vessels (G) or in the urethra (U), in particular to temporarily support the prostate (P), consisting of a thin-walled, substantially tubular body with an inlet opening (12) at one end and an outlet opening (13) at the other end, wherein the body is formed from a material reabsorbable into the patient, wherein the body has a closed outer surface (11) and outwardly-directed fixing elements are provided at the outer surface of the stent (1), **characterised in that** the fixing elements are formed on the outer surface (11) of the stent (1) so as to splay out elastically.

2. A stent according to claim 1, **characterised in that** close to each of the inlet opening (12) and the outlet opening (13) there are in each case provided three fixing elements (3) arranged through 120° in a radial plane with respect to the stent (1), both fixing element arrangements blocking opposite-directed axial directions of motion of the stent (1).

3. A stent according to claim 1 or 2, **characterised in that** laser light reflecting ultra-small particles (21) are incorporated in the reabsorbable material in a substantially uniform distribution.

4. A stent according to claim 1, 2 or 3, **characterised in that** incrustation-inhibiting and/or inflammation-inhibiting pharmaceuticals are arranged in the reabsorbable material and/or on the surface thereof, in a substantially uniform distribution.

5. A stent according to claim 4, **characterised in that** antibiotics such as e.g. fluoroquinolone and nitroxolin [sic], antifungal agents such as e.g. fluconazole, analgesics such as e.g. lidocaine and spasmolytics such as e.g. butyl scopolaminium bromide are contained as pharmaceuticals as a single substance or together.

6. A stent according to any one of the preceding claims, **characterised in that** X-ray, CT and/or NMR contrast media are embedded in the reabsorbable material in a substantially uniform distribution.

7. A stent according to any one of the preceding claims, **characterised in that** metal ions (22) are embedded in the reabsorbable material of the stent (1).

8. A stent according to any one of the preceding claims, **characterised in that** the closed outer surface (11) is externally microporous.

9. A stent according to any one of the preceding claims, **characterised in that** the reabsorbable material is formed from poly-(L)-lactide, poly-(D,L)-lactide, polyglycolic acid, polymers of maleic acid, poly-β-hydroxybutanoic acid, polydioxanone or copolymers thereof.

10. A stent according to any one of the preceding claims, **characterised in that** biocompatible plasticisers are added to the reabsorbable material.

11. A stent for maintaining the volume flow in the urethra (U) between the bladder (B) and the bladder outlet (A) according to any one of the preceding claims, **characterised in that** a funnel-like or umbrella-like widening (4) is provided at the inlet opening (12) facing the bladder (B).

12. A stent according to claim 11, **characterised in that** the widening (4) is planar and limp.

13. A stent according to claim 11 or 12, **characterised in that** the funnel-like or umbrella-like widening (4) has at least one substantially radial cut (41).

14. A stent according to claim 11, 12 or 13, **characterised in that** the funnel-like or umbrella-like widening (4) has at least one cut-out (42) substantially in the form of a segment of a circle.

15. A stent according to any one of claims 11 to 14, **characterised in that** the stent body has an external diameter of 4 to 7 mm, preferably 5 mm, as well as a length of 80 to 120 mm, preferably 100 nun.

## Revendications

1. Stent (1) pour la conservation du débit volumétrique dans des vaisseaux sanguins (G) ou dans l'urètre (U), notamment pour le support temporaire de la prostate (P), composé d'un corps de faible paroi essentiellement tubulaire avec une ouverture d'entrée (12) à une extrémité et une ouverture de sortie (13) à l'autre extrémité, le corps étant réalisé dans un matériau résorbable dans le corps du patient, le corps comprenant une enveloppe (11) fermée et des éléments de fixation dirigés vers l'extérieur étant prévus sur l'enveloppe du stent (1), **caractérisé en ce que** les éléments de fixation sont formés sur l'enveloppe (11) du stent (1) en s'écartant élastiquement.

2. Stent selon la revendication 1**, caractérisé en ce qu'**à proximité de l'ouverture d'entrée (12) et à proximité de l'ouverture de sortie (13), trois éléments de fixation (3) sont prévus disposés à 120° dans le plan radial par rapport au stent (1), les deux arrangements d'éléments de fixation bloquant des mouvements axiaux opposées du stent (1).

3. Stent selon la revendication 1 ou 2, **caractérisé en ce que** des microparticules (21) réfléchissant la lumière laser sont intégrées dans le matériau résorbable selon une répartition essentiellement homogène.

4. Stent selon la revendication 1, 2 ou 3, **caractérisé en ce que** des produits pharmaceutiques anti-incrustation et/ou anti-inflammatoires sont disposés de façon essentiellement homogène dans le matériau résorbable et/ou à sa surface.

5. Stent selon la revendication 4, **caractérisé en ce que** l'on utilise comme produits pharmaceutiques des antibiotiques tels que des fluoroquinolones et de la nitroxiline, des antimycosiques tels que par exemple du fluconazole, des analgésiques tels que de la lidocaïne et des spasmolytiques tels que par exemple du bromure de butylscopolamine, soit comme substance seule soit en combinaison.

6. Strent selon l'une des revendications précédentes, **caractérisé en ce que** des produits de contraste pour rayons X, pour CT et/ou pour RMN sont introduits de façon essentiellement homogène dans le matériau résorbable.

7. Stent selon l'une des revendications précédentes, **caractérisé en ce que** des ions métalliques (11) sont introduits dans le matériau résorbable du stent (1).

8. Stent selon l'une des revendications précédentes, **caractérisé en ce que** l'enveloppe fermée (11) est conçue du côté extérieur de façon microporeuse.

9. Stent selon l'une des revendications précédentes, **caractérisé en ce que** le matériau résorbable est fabriqué dans du poly-(L)-lactide, du poly-(DL)-lactide, de l'acide polyglycolique, de polymères de l'acide maléique, de l'acide poly-β-hydroxybutyrique, du polydioxanone, des mélanges ou des copolymères de ceux-ci.

10. Stent selon l'une des revendications précédentes, **caractérisé en ce que** des plastifiants biocompatibles sont ajoutés au matériau résorbable.

11. Stent destiné à la conservation du débit volumétrique dans l'urètre (U) entre la vessie (B) et la sortie de l'urètre (A) selon l'une des revendications précédentes, **caractérisé en ce qu'**il est prévu au niveau de l'ouverture d'entrée (12) dirigée vers la vessie (B) un élargissement (4) en forme d'entonnoir ou de parapluie.

12. Stent selon la revendication 11, **caractérisé en ce que** l'élargissement (4) est conçu en nappe et sans rigidité flexionnelle.

13. Stent selon la revendication 11 ou 12, **caractérisé en ce que** l'élargissement (4) en forme d'entonnoir ou de parapluie présente au moins une entaille (41) s'étendant essentiellement de façon radiale.

14. Stent selon l'une des revendications 11, 12 ou 13, **caractérisé en ce que** l'élargissement (4) en forme d'entonnoir ou de parapluie présente au moins une découpe (42) ayant essentiellement la forme d'un segment de cercle.

15. Stent selon l'une des revendications 11 à 14, **caractérisé en ce que** le corps de stent a un diamètre extérieur de 4 à 7 mm, de préférence de 5 mm, et une longueur de 80 à 120 mm, de préférence de 100 mm.
